# FASCICULE DE BREVET EUROPEEN

(11) **EP 4 079 586 B1**
(45) Date de publication et mention de la délivrance du brevet: **20.12.2023**
(21) Numéro de dépôt: 22168654.6
(22) Date de dépôt: 15.04.2022
(51) Int. Cl.: B60S 1/48, B60S 1/66, A61L 2/18, B60S 1/68

(54) **ENGIN ROULANT, TEL QU'UNE REMORQUE, UN VÉHICULE TRACTEUR, UN SEMI-REMORQUE, UN CAMION OU AUTRE**
ROLLENDER WAGEN, Z. B. ANHÄNGER, ZUGFAHRZEUG, SATTELSCHLEPPER, LASTWAGEN ODER ÄHNLICHES
WHEELED VEHICLE, SUCH AS A TRAILER, TOWING VEHICLE, SEMI-TRAILER, TRUCK OR OTHER

(30) Priorité: 21.04.2021 FR 2104137
(43) Date de publication de la demande: 26.10.2022
(73) Titulaire: Segasel, 85110 Chantonnay (FR)
(72) Inventeur: SELLIER, Jean-Philippe, 85110 CHANTONNAY (FR)
(74) Mandataire: Ipsilon

(56) Documents cités:
- FR-A1- 3 055 120
- FR-A1- 3 092 542
- JP-A- 2003 104 175
- JP-A- 2012 086 648
- JP-A- 2019 123 296
- US-A1- 2002 162 580

## Description

La présente invention concerne un engin roulant, tel qu'une remorque, un véhicule tracteur, un semi-remorque, un camion ou autre.

Elle concerne en particulier un engin roulant comprenant un châssis, des roues de déplacement au sol du châssis, un système de détermination de la vitesse de l'engin, une mémoire de stockage d'au moins une vitesse de consigne, une unité de pilotage et une installation embarquée de désinfection des roues et du dessous du châssis de l'engin, ladite installation comprenant un réservoir de fluide de désinfection, une pompe de distribution du fluide de désinfection, et un circuit de distribution de fluide équipé en sortie de buses orientées en direction des roues et du dessous du châssis de l'engin.

De nombreux engins roulants sont, pour des raisons diverses et variées, amenés à circuler entre deux zones dites sensibles, telles que par exemple des bâtiments d'élevage d'exploitations différentes. Le risque est que ce déplacement entraîne une contamination par l'un des sites de l'autre site. Pour éviter cela, des solutions permettant en particulier de désinfecter les roues et le dessous du châssis encore appelé soubassement de l'engin roulant au moins juste avant de pénétrer dans une zone sensible à protéger ont été imaginées. Toutefois, les solutions développées à ce jour ne permettent pas de garantir une désinfection fiable et efficace.

Le document FR-3.055.120 divulgue un engin roulant comprenant un châssis, des roues de déplacement au sol du châssis, un système de détermination de la vitesse de l'engin, une mémoire de stockage d'au moins une vitesse de consigne, une unité de pilotage et une installation embarquée de désinfection des roues et du dessous du châssis de l'engin, ladite installation comprenant un réservoir de fluide de désinfection, une pompe de distribution du fluide de désinfection et un circuit de distribution de fluide équipé en sortie de buses dont au moins certaines sont orientées en direction des roues de l'engin. L'engin comprend encore une source de fluide sous pression.

Un but de l'invention est de proposer un engin dont la conception permet de garantir une désinfection fiable et efficace au niveau des roues et du dessous du châssis de l'engin à un coût raisonnable.

À cet effet, l'invention a pour objet un engin roulant comprenant un châssis, des roues de déplacement au sol du châssis, un système de détermination de la vitesse de l'engin, une mémoire de stockage d'au moins une vitesse de consigne, une unité de pilotage et une installation embarquée de désinfection des roues et du dessous du châssis de l'engin, ladite installation comprenant un réservoir de fluide de désinfection, une pompe de distribution du fluide de désinfection, et un circuit de distribution de fluide équipé en sortie de buses dont au moins certaines sont orientées en direction des roues de l'engin, caractérisé en ce que l'engin comprend une source de fluide sous pression, un mode de fonctionnement dit de désinfection activable/désactivable et un organe à actionnement manuel d'activation dudit mode de fonctionnement, en ce que le circuit de distribution de fluide est raccordé, par une première liaison sur laquelle est disposée la pompe de l'installation, au réservoir de fluide de désinfection et, par une seconde liaison équipée d'un organe d'obturation monté mobile entre une position ouverte et une position fermée, à la source de fluide sous pression, en ce que le mode de fonctionnement de désinfection est un mode de fonctionnement activable/désactivable en fonction au moins des données fournies par le système de détermination de la vitesse, en ce que l'unité de pilotage est configurée pour, à l'état actionné manuellement de l'organe d'activation, commander l'activation du mode de fonctionnement de désinfection, lorsque la vitesse de l'engin est comprise à l'intérieur d'une plage de vitesses de consigne prédéfinie et en ce que l'unité de pilotage est configurée pour, à l'état activé du mode de fonctionnement de désinfection, commander la pompe et l'organe d'obturation suivant un cycle de désinfection comprenant au moins une première phase d'actionnement de la pompe pour une alimentation du circuit de distribution de fluide en fluide de désinfection et une deuxième phase d'actionnement de l'organe d'obturation dans le sens d'une ouverture de la seconde liaison pour une alimentation, du circuit de distribution de fluide prérempli en fluide de désinfection, en fluide sous pression, ce fluide sous pression faisant fonction de fluide propulseur du fluide de désinfection à travers les buses. L'activation du mode de désinfection en fonction au moins des données fournies par le système de détermination de la vitesse permet de garantir une désinfection en roulant, cette désinfection s'opérant généralement à l'intérieur d'une plage de vitesses de consigne de préférence paramétrable et généralement comprise entre 1 et 10 km/h. Cette désinfection en roulant permet d'utiliser la roue comme moyen de dispersion du fluide de désinfection sur le châssis. Cette conception permet de garantir une désinfection de la roue sur au moins un tour de rotation complet, généralement sur plusieurs tours de rotation de la roue. La mise en oeuvre, pendant le cycle de désinfection, d'un pré-remplissage en liquide de désinfection du circuit de distribution de fluide avant propulsion dudit liquide de désinfection à travers les buses à l'aide d'un fluide propulseur permet de générer un brouillard qui s'avère extrêmement efficace pour la désinfection par comparaison à un jet de fluide tout en garantissant la quantité et la durée de pulvérisation, le circuit de distribution étant utilisé comme zone de stockage avant pulvérisation. Il en résulte une simplicité de conception. La possibilité d'utiliser ce fluide sous pression en dehors du cycle de désinfection permet d'éviter tout colmatage des buses, ce qui est un avantage essentiel lorsque l'engin roulant est amené à circuler par exemple entre des exploitations agricoles ou entre deux carrières.

Selon un mode de réalisation de l'invention, l'engin comprend un deuxième mode de fonctionnement dit de décolmatage des buses activable/désactivable, l'unité de pilotage est configurée pour commander automatiquement l'activation ou la désactivation du deuxième mode de fonctionnement en fonction au moins des données fournies par le système de détermination de la vitesse, et l'unité de pilotage est configurée pour, à l'état activé du deuxième mode de fonctionnement, commander l'ouverture et la fermeture de l'organe d'obturation suivant un séquençage prédéfini. Ce deuxième mode de fonctionnement de décolmatage des buses au cours duquel un fluide sous pression, en particulier un gaz sous pression, tel que de l'air comprimé, peut être envoyé dans les buses permet de manière simple et à faible coût un décolmatage des buses sans nuire à l'efficacité de la désinfection. Le fait d'asservir l'activation et la désactivation du mode de fonctionnement de désinfection et du deuxième mode de fonctionnement de décolmatage à la vitesse de l'engin permet d'éviter une perturbation du cycle de désinfection par une opération de décolmatage et garantit un décolmatage dans des conditions où aucune personne ne se trouve à proximité de l'engin roulant. Ce décolmatage des buses apte à générer une vidange en fluide de désinfection du circuit de distribution permet également de limiter les risques de gel à l'intérieur du circuit de distribution de fluide.

Selon un mode de réalisation de l'invention, l'unité de pilotage est configurée pour commander automatiquement l'activation du deuxième mode de fonctionnement lorsque la vitesse de l'engin est supérieure à une vitesse de consigne seuil, ladite vitesse de consigne seuil étant supérieure à la plage de vitesses de consigne prédéfinie. Cette conception d'une vitesse de consigne seuil supérieure à la plage de vitesses de consigne prédéfinie d'activation du mode de fonctionnement de désinfection permet de ne pas perturber le cycle de désinfection par un décolmatage des buses.

Selon un mode de réalisation de l'invention, l'unité de pilotage est configurée pour, à l'état activé du mode de fonctionnement de désinfection, commander automatiquement la désactivation du mode de fonctionnement de désinfection, lorsque la vitesse de l'engin est en dehors de la plage de vitesses de consigne prédéfinie. Cette disposition permet de garantir une efficacité du cycle de désinfection.

Selon un mode de réalisation de l'invention, le cycle de désinfection comprend, après la deuxième phase d'actionnement de l'organe d'obturation dans le sens d'une ouverture de la seconde liaison, une troisième phase d'actionnement de l'organe d'obturation dans le sens d'une fermeture de la seconde liaison, et l'unité de pilotage est configurée pour commander automatiquement la désactivation du mode de fonctionnement de désinfection à l'issue de la phase d'actionnement de l'organe d'obturation dans le sens d'une fermeture de la seconde liaison. Le mode de fonctionnement de désinfection est ainsi automatiquement désactivé une fois un cycle de désinfection réalisé.

Selon un mode de réalisation de l'invention, l'engin comprend un système de géolocalisation, une horloge et une deuxième mémoire de stockage de données fournies par l'horloge et le système de géolocalisation et l'unité de pilotage est configurée pour, à l'état activé du mode de fonctionnement de désinfection, commander la mémorisation des données fournies par l'horloge et le système de géolocalisation en fonction au moins de l'état ouvert/fermé de l'organe d'obturation et/ou de l'état marche/arrêt de la pompe. Cette conception permet de tenir un journal des cycles de désinfection. Ce journal peut être adressé à un terminal déporté ou téléchargé.

Selon un mode de réalisation de l'invention, le réservoir de fluide de désinfection est équipé d'au moins un organe de chauffage. Il en résulte une efficacité accrue de la désinfection.

Selon un mode de réalisation de l'invention, le système de détermination de la vitesse de l'engin est un tachymètre.

Selon un mode de réalisation de l'invention, lesdites première et seconde liaisons sont équipées chacune d'un clapet anti-retour s'étendant entre le circuit de distribution et la pompe pour la première liaison et entre le circuit de distribution et l'organe d'obturation pour la seconde liaison. Cette conception se caractérise par sa simplicité.

Selon un mode de réalisation de l'invention, lesdites première et seconde liaisons sont équipées chacune d'un capteur de pression s'étendant entre le circuit de distribution et la pompe pour la première liaison et entre le circuit de distribution et l'organe d'obturation pour la seconde liaison et l'unité de pilotage est configurée pour commander le passage de l'état actif à l'état inactif du mode de fonctionnement de désinfection en fonction des données fournies par lesdits capteurs de pression. Cette disposition permet à nouveau de garantir une désinfection efficace et de détecter une éventuelle défaillance de l'installation de désinfection.

### Brève description des dessins

L'invention sera bien comprise à la lecture de la description suivante d'exemples de réalisation, en référence aux dessins annexés dans lesquels :
[Fig. 1] représente une vue schématique d'un engin conforme à l'invention,
[Fig. 2] représente une vue schématique de l'installation de désinfection,
[Fig. 3] représente sous forme de blocs les étapes d'un cycle de désinfection.

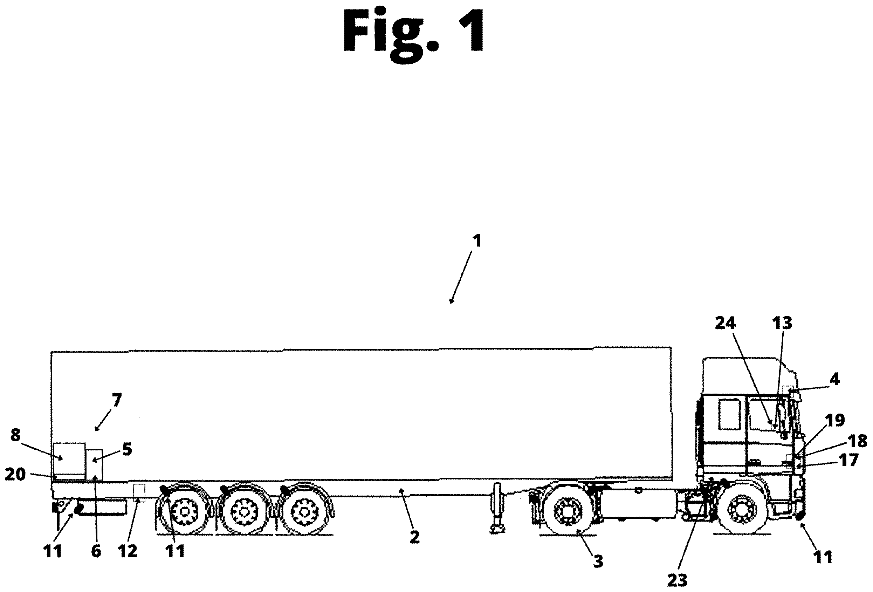

Comme mentionné ci-dessus, l'invention a pour objet un engin 1 roulant, tel que représenté par exemple à la figure 1, cet engin 1 roulant pouvant être amené à circuler entre des zones sensibles ne devant pas faire l'objet d'une contamination entre elles.

L'engin 1 roulant, tel que représenté à la figure 1, est un semi-remorque avec un véhicule tracteur et une remorque, mais aurait pu de manière équivalente être formé par tout autre type d'engin roulant sans sortir du cadre de l'invention.

Cet engin 1 roulant comprend donc un châssis 2 qui peut être en plusieurs parties, et des roues 3 de déplacement au sol du châssis 2.

Cet engin 1 roulant comprend généralement une cabine de pilotage, à l'intérieur de laquelle un conducteur de l'engin est apte à prendre place.

Cet engin 1 comprend encore un système 4 de détermination de la vitesse de l'engin 1.

Dans les exemples représentés, le système 4 de détermination de la vitesse de l'engin est un tachymètre, mais tout capteur de vitesse autre et/ou tout système de géolocalisation, en particulier tout appareil avec une antenne de géolocalisation, telle qu'une antenne GNSS (acronyme de géolocalisation en navigation par un système de stallites), est approprié pour déterminer la vitesse de déplacement de l'engin 1 roulant.

Cet engin 1 roulant comprend encore une mémoire 5 de stockage d'au moins une vitesse de consigne. Le rôle de la ou les vitesses de consigne sera décrit ci-après.

L'engin 1 roulant comprend également une unité 6 de pilotage. Cette unité 6 de pilotage est notamment configurée pour acquérir des données du système 4 de détermination de la vitesse de l'engin 1 et pour les comparer avec au moins l'une des vitesses de consigne mémorisées.

L'unité 6 de pilotage se présente sous la forme d'un système électronique et informatique qui comprend par exemple un microprocesseur et une mémoire de travail. Selon un aspect particulier, l'unité de pilotage peut se présenter sous la forme d'un automate programmable. Autrement dit, les fonctions et étapes décrites peuvent être mises en oeuvre sous forme de programme informatique ou via des composants matériels (p. ex. des réseaux de portes programmables). En particulier, les fonctions et étapes opérées par l'unité de pilotage ou ses modules peuvent être réalisées par des jeux d'instructions ou modules informatiques implémentés dans un processeur ou contrôleur ou être réalisées par des composants électroniques dédiés ou des composants de type FPGA ou ASIC. Il est aussi possible de combiner des parties informatiques et des parties électroniques. Lorsqu'il est précisé que l'unité ou des moyens ou modules de ladite unité sont configurés pour réaliser une opération donnée, cela signifie que l'unité comprend des instructions informatiques et les moyens d'exécution correspondants qui permettent de réaliser ladite opération et/ou que l'unité comprend des composants électroniques correspondants.

L'engin 1 roulant comprend encore une installation 7 embarquée de désinfection des roues 3 et du dessous du châssis de l'engin. Cette installation 7 comprend un réservoir 8 de fluide de désinfection qui, dans l'exemple représenté, est disposée à l'arrière de la remorque de l'engin 1 roulant. Ce fluide de désinfection est un liquide qui contient au moins un désinfectant tel qu'un agent bactéricide, un agent virucide ou équivalent.

Cette installation 7 de désinfection comprend encore une pompe 9 de distribution de fluide de désinfection apte à être pilotée par l'unité 6 de pilotage et un circuit 10 de distribution de fluide équipé en sorties de buses 11 dont au moins certaines sont orientées en direction des roues 3 de l'engin 1.

Ainsi, dans l'exemple représenté à la figure 1, un certain nombre de buses sont disposées sur les garde-boues des roues de l'engin et sont orientées en direction des roues de l'engin. Des buses 11 sont également disposées à l'avant du véhicule tracteur de la remorque et sont orientées vers le dessous du châssis de l'engin. D'autres buses 11 sont disposées au niveau de l'arrière de l'engin 1 et sont orientées en direction d'une bavette disposée au niveau du pare-chocs arrière de la remorque de l'engin 1 roulant. Chaque buse présente un orifice calibré. Du fait que l'engin se déplace, l'ensemble de la partie inférieure de l'engin traverse le brouillard pulvérisé par les buses sur les roues.

L'engin 1 comprend encore une source 12 de fluide sous pression raccordable à un générateur de fluide sous pression, tel qu'un compresseur 23, embarqué sur l'engin.

La source 12 de fluide sous pression, en particulier de gaz sous pression, peut être formée, comme dans l'exemple représenté, par une réserve d'air comprimé.

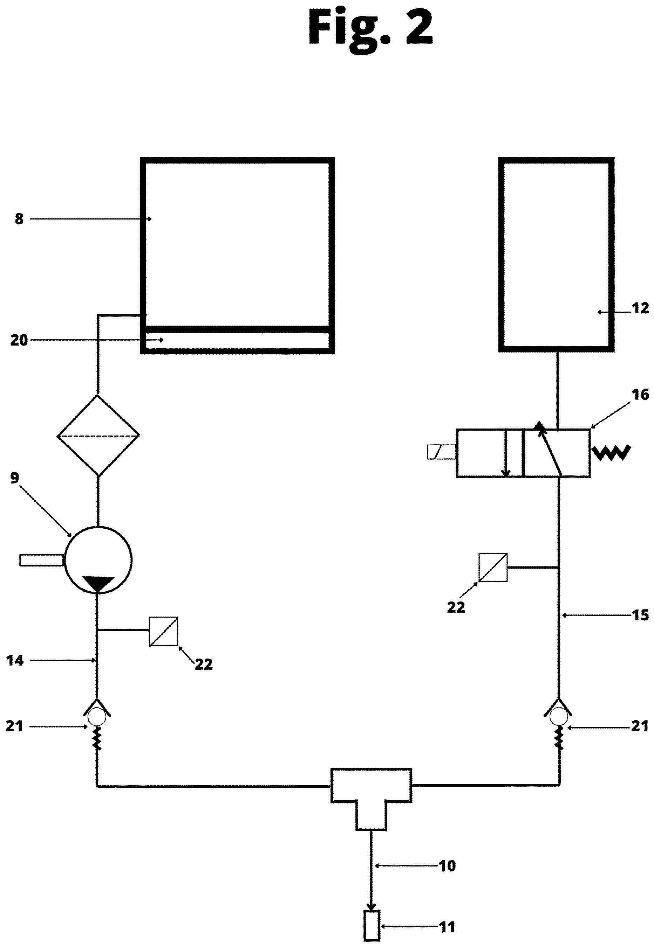

Comme illustré à la figure 2, le circuit 10 de distribution est raccordé par une première liaison 14 fluidique sur laquelle est disposée la pompe 9 de l'installation 7 au réservoir 8 de fluide de désinfection, et par une seconde liaison 15 équipée d'un organe d'obturation 16 monté mobile entre une position ouverte et une position fermée à la source 12 de fluide sous pression.

L'organe d'obturation 16 est, dans l'exemple représenté, une électrovalve apte à être pilotée pour son passage de la position fermée à la position ouverte ou inversement, par l'unité 6 de pilotage.

Dans l'exemple représenté à la figure 2, un filtre à eau est disposé en amont de la pompe 9, entre la pompe 9 et le réservoir 8 de fluide de désinfection.

La première liaison 14 est équipée d'un clapet anti-retour 21 s'étendant entre le circuit 10 de distribution et la pompe 9. La seconde liaison 15 est équipée d'un clapet anti-retour 21 s'étendant entre le circuit 10 de distribution et l'organe d'obturation 16.

La première liaison 14 est également équipée d'un capteur de pression 22 s'étendant entre le circuit 10 de distribution et la pompe 9, en l'occurrence entre la pompe 9 et le clapet 21 anti-retour. De manière similaire, la seconde liaison 15 est équipée d'un capteur de pression 22 s'étendant entre le circuit 10 de distribution et l'organe d'obturation 16, en l'occurrence ici entre l'organe d'obturation 16 et le clapet 21 anti-retour de la seconde liaison 15.

Dans les exemples représentés, le réservoir 8 de fluide de désinfection est équipé d'un organe de chauffage 20 formé ici par une résistance électrique. Cet organe de chauffage 20 peut être logé à l'intérieur dudit réservoir 8 de fluide de désinfection ou à l'extérieur du réservoir 8 de fluide de désinfection comme représenté.

L'engin 1 comprend encore un mode de fonctionnement dit de désinfection activable/désactivable et un organe 13 à actionnement manuel d'activation dudit mode de fonctionnement de désinfection.

Dans les exemples représentés, cet organe 13 d'activation actionnable manuellement par le conducteur de l'engin 1 roulant est un bouton disposé sur un écran 24 disposé à l'intérieur de la cabine de pilotage de l'engin 1, cet écran 24 formant une interface homme-machine.

L'appui sur le bouton permet de commander l'activation du mode de fonctionnement de désinfection. Bien évidemment, ce bouton aurait pu être remplacé par tout autre type d'organe de commande, tel qu'un levier ou autre, sans sortir du cadre de l'invention.

Le mode de fonctionnement de désinfection est un mode de fonctionnement activable/désactivable en fonction, au moins, des données fournies par le système 4 de détermination de la vitesse. En particulier, l'unité 6 de pilotage est configurée pour, à l'état actionné manuellement de l'organe 13 d'activation, commander l'activation du mode de fonctionnement de désinfection lorsque la vitesse de l'engin 1 roulant est comprise à l'intérieur d'une plage de vitesses de consigne prédéfinie. Cette plage de vitesses de consigne est généralement comprise entre 1 et 10 km/h. La mémoire 5 de stockage d'au moins une vitesse de consigne peut donc mémoriser les valeurs de 1 et de 10 km/h dans ce cas.

L'unité 6 de pilotage est configurée pour, à l'état activé du mode de fonctionnement de désinfection, commander automatiquement la désactivation du mode de fonctionnement de désinfection lorsque la vitesse de l'engin 1 est en dehors de la plage de vitesses de consigne prédéfinie.

L'engin 1 peut être équipé d'un dispositif d'émission d'un signal d'alerte, tel qu'un signal lumineux sonore ou vibratoire, dont au moins une partie est disposée dans la cabine de pilotage et la désactivation du mode de fonctionnement de désinfection peut s'accompagner de l'émission d'un tel signal lumineux, prenant la forme par exemple d'un témoin lumineux disposé sur l'écran 24 d'affichage.

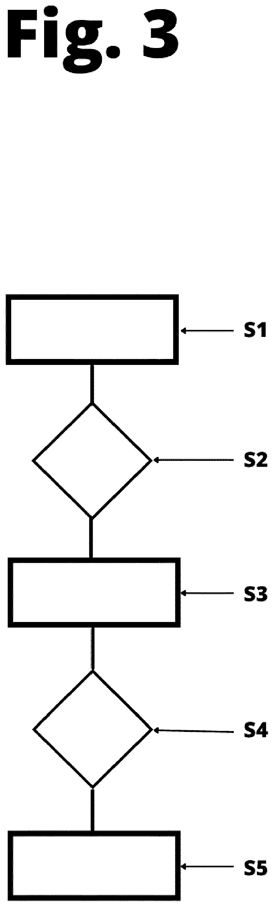

L'unité 6 de pilotage est configurée pour, à l'état activé du mode de fonctionnement de désinfection, commander la pompe 9 de la première liaison 14 et l'organe d'obturation 16 de la seconde liaison 15, suivant un cycle de désinfection tel qu'illustré à la figure 3.

Ce cycle de désinfection comprend au moins une première phase d'actionnement de la pompe 9 pour une alimentation du circuit 10 de distribution de fluide en fluide de désinfection, suivie d'une deuxième phase d'actionnement de l'organe d'obturation 16 dans le sens d'une ouverture de la seconde liaison 15 pour une alimentation, du circuit 10 de distribution prérempli en fluide de désinfection, en fluide sous-pression. Ce fluide sous-pression fait fonction de fluide propulseur du fluide de désinfection à travers les buses 11.

La deuxième phase d'actionnement de l'organe d'obturation 16 dans le sens d'une ouverture de la seconde liaison 15 est suivie d'une troisième phase d'actionnement de l'organe d'obturation 16 dans le sens d'une fermeture de la seconde liaison 15, et l'unité 6 de pilotage est configurée pour commander automatiquement la désactivation du mode de fonctionnement de désinfection à l'issue de la troisième phase d'actionnement de l'organe d'obturation 16 dans le sens d'une fermeture de la seconde liaison 15.

Dans le détail, et comme illustré à la figure 3, à l'étape S1, la pompe 9 est actionnée pour permettre le remplissage du circuit 10 de distribution de fluide qui s'étend entre les clapets 21 anti-retour et les buses. Cet actionnement de la pompe s'opère pendant une durée déterminée.

À l'étape S2, l'unité 6 de pilotage compare la valeur de pression fournie par le capteur de pression 22 équipant la première liaison 14, avec une valeur de consigne de pression prédéterminée, pour vérifier que l'installation dite de désinfection n'est pas défaillante. Si le résultat de la comparaison fait apparaître une défaillance, le mode de fonctionnement de désinfection est désactivé et un signal d'alerte est émis. La désactivation du mode de fonctionnement de désinfection entraîne en parallèle l'arrêt du cycle de désinfection.

Si le résultat de la comparaison ne fait apparaître aucune défaillance, à l'étape S3, l'organe d'obturation 16 de la liaison 15 est amené en position ouverte pour alimenter en fluide sous pression le circuit 10 de distribution pendant une durée déterminée, afin d'assurer la projection de fluide de désinfection hors des buses 11, en direction au moins des roues 3 de l'engin 1 roulant.

À l'étape S4, l'installation 6 de pilotage compare la valeur de pression fournie par le capteur de pression 22 équipant la seconde liaison 15 avec une valeur de consigne de pression prédéterminée pour vérifier que l'installation de désinfection n'est pas défaillante. Si le résultat de la comparaison fait apparaître une défaillance, le mode de fonctionnement de désinfection est à nouveau désactivé et un signal d'alerte est émis. À nouveau, le cycle de désinfection est interrompu. Si au contraire, le résultat de la comparaison ne fait apparaître aucune défaillance, à l'étape S5, la pulvérisation se poursuit pendant une durée déterminée, jusqu'à fermeture de l'organe d'obturation 16 de la seconde liaison 15 et désactivation automatique du mode de fonctionnement de désinfection. Le cycle de désinfection est achevé et un nouvel actionnement de l'organe d'activation sera nécessaire pour lancer un nouveau cycle de désinfection.

Bien évidemment, pendant la durée du cycle de désinfection tel que décrit ci-dessus, l'unité de pilotage compare la vitesse de l'engin avec la plage de vitesse de consigne prédéfinie pour désactiver le mode de fonctionnement de désinfection si la vitesse de l'engin est en dehors de la plage de vitesses de consigne prédéfinie.

La désactivation du mode de fonctionnement de désinfection entraîne une interruption du cycle de désinfection.

L'engin 1 comprend encore un deuxième mode de fonctionnement activable/désactivable dit de décolmatage des buses 11. L'unité 6 de pilotage est configurée pour commander automatiquement l'activation ou la désactivation du deuxième mode de fonctionnement en fonction au moins des données fournies par le système 4 de détermination de la vitesse, et l'unité de pilotage est configurée pour, à l'état activé du deuxième mode de fonctionnement, commander l'ouverture et la fermeture de l'organe d'obturation 16, suivant un séquençage prédéfini.

L'unité 6 de pilotage est configurée pour commander automatiquement l'activation du deuxième mode de fonctionnement lorsque la vitesse de l'engin 1 est supérieure à une vitesse de consigne seuil, ladite vitesse de consigne seuil étant supérieure à la plage de vitesses de consigne prédéfinie.

En pratique, la commande automatique de l'activation du deuxième mode de fonctionnement s'opère lorsque la vitesse de l'engin 1 roulant est supérieure à 40 km/h. Le séquençage prédéfini de l'organe d'obturation 16 comprend au moins une phase d'ouverture de l'organe d'obturation 16 et une phase de fermeture de l'organe d'obturation 16. Ces phases d'ouverture et de fermeture sont généralement de durée prédéfinie.

Dans l'exemple représenté, lorsque la vitesse est supérieure à 40 km/h, l'organe d'obturation 16 de la seconde liaison 15 est ouvert pendant cinq secondes, puis fermé pendant dix secondes. Ces étapes d'ouverture et de fermeture de l'organe d'obturation 16 sont répétées quatre fois avant de faire une pause de cinq minutes, puis de reprendre le cycle d'ouverture/fermeture de l'organe d'obturation 16 tel que décrit ci-dessus. Ce processus est poursuivi tant que la vitesse de l'engin est supérieure à la vitesse de consigne prédéfinie.

Pour parfaire l'ensemble, l'engin 1 comprend un système de géolocalisation 17, une horloge 18 et une deuxième mémoire 19 de stockage de données fournies par l'horloge 18 et le système de géolocalisation 17.

L'unité 6 de pilotage est configurée pour, à l'état activé du mode de fonctionnement de désinfection, commander la mémorisation des données fournies par l'horloge 18 et le système de géolocalisation 17 en fonction au moins de l'état ouvert ou fermé de l'organe 16 d'obturation et/ou de l'état marche/arrêt de la pompe 9.

La deuxième mémoire 14 de stockage et la mémoire 5 de stockage de vitesse de consigne peuvent être communes et être formées par une seule et même mémoire de stockage.

Les données ainsi recueillies permettent d'établir un journal de bord des cycles de désinfection et de décolmatage. Ces données peuvent être téléchargées ou adressées à l'aide d'un émetteur sans fil à un terminal déporté, pour permettre un contrôle à distance des opérations opérées.

En particulier, la commande d'un tel engin est simple. Lorsque le conducteur de l'engin souhaite procéder à un cycle de désinfection, il réduit la vitesse de l'engin jusqu'à atteindre la plage de vitesses de consigne prédéfinie. Une indication lumineuse sur l'écran d'affichage peut l'aider à respecter cette plage de vitesses. Il actionne alors l'organe 13 d'activation du mode de fonctionnement de désinfection. Un cycle de désinfection tel que décrit ci-dessus peut s'opérer. Ce cycle de désinfection peut être interrompu à tout moment par désactivation du mode de fonctionnement de désinfection, si les conditions de vitesse de l'engin ou de pression ne sont pas remplies. Un signal d'alerte, généralement lumineux, est alors émis.

Dès qu'un cycle de désinfection est achevé, le mode de fonctionnement de désinfection est désactivé automatiquement. Dès que l'engin 1 roule à une vitesse supérieure à une vitesse de consigne prédéfinie, en l'occurrence ici 40 km/h, le deuxième mode de fonctionnement de décolmatage de buses est activé.

## Revendications

1. Engin (1) roulant comprenant un châssis (2), des roues (3) de déplacement au sol du châssis (2), un système (4) de détermination de la vitesse de l'engin (1), une mémoire (5) de stockage d'au moins une vitesse de consigne, une unité (6) de pilotage et une installation (7) embarquée de désinfection des roues (3) et du dessous du châssis (2) de l'engin (1), ladite installation (7) comprenant un réservoir (8) de fluide de désinfection, une pompe (9) de distribution du fluide de désinfection, et un circuit (10) de distribution de fluide équipé en sortie de buses (11) dont au moins certaines sont orientées en direction des roues (3) de l'engin (1), l'engin (1) comprenant une source (12) de fluide sous pression, un mode de fonctionnement dit de désinfection activable/désactivable et un organe (13) à actionnement manuel d'activation dudit mode de fonctionnement, le circuit (10) de distribution de fluide étant raccordé, par une première liaison (14) sur laquelle est disposée la pompe (9) de l'installation (7), au réservoir (8) de fluide de désinfection et, par une seconde liaison (15) équipée d'un organe d'obturation (16) monté mobile entre une position ouverte et une position fermée, à la source (12) de fluide sous pression, **caractérisé en ce que** le mode de fonctionnement de désinfection est un mode de fonctionnement activable/désactivable en fonction au moins des données fournies par le système (4) de détermination de la vitesse, et **en ce que** l'unité (6) de pilotage est configurée pour, à l'état actionné manuellement de l'organe (13) d'activation, commander l'activation du mode de fonctionnement de désinfection, lorsque la vitesse de l'engin (1) est comprise à l'intérieur d'une plage de vitesses de consigne prédéfinie et **en ce que** l'unité (6) de pilotage est configurée pour, à l'état activé du mode de fonctionnement de désinfection, commander la pompe (9) et l'organe d'obturation (16) suivant un cycle de désinfection comprenant au moins une première phase d'actionnement de la pompe (9) pour une alimentation du circuit (10) de distribution de fluide en fluide de désinfection et une deuxième phase d'actionnement de l'organe d'obturation (16) dans le sens d'une ouverture de la seconde liaison (15) pour une alimentation, du circuit (10) de distribution de fluide prérempli en fluide de désinfection, en fluide sous pression, ce fluide sous pression faisant fonction de fluide propulseur du fluide de désinfection à travers les buses (11).

2. Engin (1) roulant selon la revendication 1, **caractérisé en ce que** l'engin (1) comprend un deuxième mode de fonctionnement dit de décolmatage des buses (11) activable/désactivable, **en ce que** l'unité (6) de pilotage est configurée pour commander automatiquement l'activation ou la désactivation du deuxième mode de fonctionnement en fonction au moins des données fournies par le système (4) de détermination de la vitesse, et **en ce que** l'unité (6) de pilotage est configurée pour, à l'état activé du deuxième mode de fonctionnement, commander l'ouverture et la fermeture de l'organe (16) d'obturation suivant un séquençage prédéfini.

3. Engin (1) roulant selon la revendication 2, **caractérisé en ce que** l'unité (6) de pilotage est configurée pour commander automatiquement l'activation du deuxième mode de fonctionnement lorsque la vitesse de l'engin (1) est supérieure à une vitesse de consigne seuil, ladite vitesse de consigne seuil étant supérieure à la plage de vitesses de consigne prédéfinie.

4. Engin (1) roulant selon l'une des revendications 1 à 3, **caractérisé en ce que** l'unité (6) de pilotage est configurée pour, à l'état activé du mode de fonctionnement de désinfection, commander automatiquement la désactivation du mode de fonctionnement de désinfection, lorsque la vitesse de l'engin (1) est en dehors de la plage de vitesses de consigne prédéfinie.

5. Engin (1) roulant selon l'une des revendications 1 à 4, **caractérisé en ce que** le cycle de désinfection comprend, après la deuxième phase d'actionnement de l'organe d'obturation (16) dans le sens d'une ouverture de la seconde liaison (15), une troisième phase d'actionnement de l'organe d'obturation (16) dans le sens d'une fermeture de la seconde liaison (15), et **en ce que** l'unité (6) de pilotage est configurée pour commander automatiquement la désactivation du mode de fonctionnement de désinfection à l'issue de la phase d'actionnement de l'organe d'obturation (16) dans le sens d'une fermeture de la seconde liaison (15).

6. Engin (1) roulant selon l'une des revendications 1 à 5, **caractérisé en ce que** l'engin (1) comprend un système de géolocalisation (17), une horloge (18) et une deuxième mémoire (19) de stockage de données fournies par l'horloge (18) et le système de géolocalisation (17) et **en ce que** l'unité (6) de pilotage est configurée pour, à l'état activé du mode de fonctionnement de désinfection, commander la mémorisation des données fournies par l'horloge (18) et le système de géolocalisation (17) en fonction au moins de l'état ouvert/fermé de l'organe d'obturation (16) et/ou de l'état marche/arrêt de la pompe (9).

7. Engin (1) roulant selon l'une des revendications 1 à 6, **caractérisé en ce que** le réservoir (8) de fluide de désinfection est équipé d'au moins un organe de chauffage (20).

8. Engin (1) roulant selon l'une des revendications 1 à 7, **caractérisé en ce que** le système (4) de détermination de la vitesse de l'engin est un tachymètre.

9. Engin (1) roulant selon l'une des revendications 1 à 8, **caractérisé en ce que** lesdites première et seconde liaisons (14, 15) sont équipées chacune d'un clapet anti-retour (21) s'étendant entre le circuit (10) de distribution et la pompe (9) pour la première liaison (14) et entre le circuit (10) de distribution et l'organe d'obturation (16) pour la seconde liaison (15).

10. Engin (1) roulant selon l'une des revendications 1 à 9, **caractérisé en ce que** lesdites première et seconde liaisons (14, 15) sont équipées chacune d'un capteur de pression (22) s'étendant entre le circuit (10) de distribution et la pompe (9) pour la première liaison (14) et entre le circuit (10) de distribution et l'organe d'obturation (16) pour la seconde liaison (15) et **en ce que** l'unité (6) de pilotage est configurée pour commander le passage de l'état actif à l'état inactif du mode de fonctionnement de désinfection en fonction des données fournies par lesdits capteurs de pression (22).

## Patentansprüche

1. Rollender Wagen (1), umfassend ein Fahrgestell (2), Räder (3) für die Fortbewegung des Fahrgestells (2) auf dem Boden, ein System (4) zur Bestimmung der Geschwindigkeit des Wagens (1), einen Speicher (5) zum Speichern von mindestens einer Sollgeschwindigkeit, eine Steuereinheit (6) und eine On-Board-Desinfektionsanlage (7) der Räder (3) und der Unterseite des Fahrgestells (2) des Wagens (1), wobei die Anlage (7) einen Desinfektionsfluid-Vorratsbehälter (8), eine Verteilerpumpe (9) des Desinfektionsfluids und einen Fluidverteilerkreis (10) umfasst, der am Ausgang mit Düsen (11) ausgestattet ist, von denen mindestens einige in Richtung der Räder (3) des Wagens (1) gerichtet sind, wobei der Wagen (1) eine Fluidquelle (12) unter Druck, einen aktivierbaren/deaktivierbaren Desinfektions-Betriebsmodus und eine Einrichtung (13) zur manuellen Betätigung der Aktivierung des Betriebsmodus umfasst, wobei der Fluidverteilerkreis (10) mittels einer ersten Verbindung (14), auf der die Pumpe (9) der Anlage (7) angeordnet ist, am Desinfektionsfluid-Vorratsbehälter (8) und mittels einer zweiten Verbindung (15), die mit einem Verschlussorgan (16) ausgestattet ist, das zwischen einer geöffneten Position und einer geschlossenen Position beweglich angebracht ist, an der Fluidquelle (12) unter Druck angeschlossen ist, **dadurch gekennzeichnet, dass** der Desinfektions-Betriebsmodus ein in Abhängigkeit von mindestens den vom System (4) zur Bestimmung der Geschwindigkeit bereitgestellten Daten aktivierbarer/deaktivierbarer Betriebsmodus ist und dass die Steuereinheit (6) dazu ausgelegt ist, damit im manuell betätigten Zustand der Aktivierungseinrichtung (13) die Aktivierung des Desinfektions-Betriebsmodus befohlen wird, wenn sich die Geschwindigkeit des Wagens (1) innerhalb eines vorher festgelegten Sollgeschwindigkeitsbereichs befindet und dass die Steuereinheit (6) dazu ausgelegt ist, im aktivierten Zustand des Desinfektions-Betriebsmodus der Pumpe (9) und dem folgenden Verschlussorgan (16) einen Desinfektionszyklus zu befehlen, der mindestens eine erste Betätigungsphase der Pumpe (9) für eine Versorgung des Fluidverteilerkreises (10) mit Desinfektionsfluid und eine zweite Betätigungsphase des Verschlussorgans (16) im Sinn einer Öffnung der zweiten Verbindung (15) für eine Versorgung des mit Desinfektionsfluid vorgefüllten Fluidverteilerkreises (10) mit Fluid unter Druck umfasst, wobei dieses Fluid unter Druck als Treibfluid des Desinfektionsfluids durch die Düsen (11) dient.

2. Rollender Wagen (1) nach Anspruch 1, **dadurch gekennzeichnet, dass** der Wagen (1) einen als Durchspülen der Düsen (11) bezeichneten aktivierbaren/deaktivierbaren zweiten Betriebsmodus umfasst, dass die Steuereinheit (6) dazu ausgelegt ist, die Aktivierung oder die Deaktivierung des zweiten Betriebsmodus in Abhängigkeit von mindestens den vom System (4) zur Bestimmung der Geschwindigkeit bereitgestellten Daten automatisch zu befehlen und dass die Steuereinheit (6) dazu ausgelegt ist, im aktivierten Zustand des zweiten Betriebsmodus das Öffnen und das Schließen des Verschlussorgans (16) gemäß einer vorher festgelegten Sequenzfolge zu befehlen.

3. Rollender Wagen (1) nach Anspruch 2, **dadurch gekennzeichnet, dass** die Steuereinheit (6) dazu ausgelegt ist, die Aktivierung des zweiten Betriebsmodus automatisch zu befehlen, wenn die Geschwindigkeit des Wagens (1) über einer Grenz-Sollgeschwindigkeit liegt, wobei die Grenz-Sollgeschwindigkeit höher als der vorher festgelegte Sollgeschwindigkeitsbereich ist.

4. Rollender Wagen (1) nach einem der Ansprüche 1 bis 3, **dadurch gekennzeichnet, dass** die Steuereinheit (6) dazu ausgelegt ist, im aktivierten Zustand des Desinfektions-Betriebsmodus die Deaktivierung des Desinfektions-Betriebsmodus automatisch zu befehlen, wenn die Geschwindigkeit des Wagens (1) außerhalb des vorher festgelegten Sollgeschwindigkeitsbereichs liegt.

5. Rollender Wagen (1) nach einem der Ansprüche 1 bis 4, **dadurch gekennzeichnet, dass** der Desinfektionszyklus nach der zweiten Betätigungsphase des Verschlussorgans (16) im Sinn einer Öffnung der zweiten Verbindung (15) eine dritte Betätigungsphase des Verschlussorgans (16) im Sinn eines Schließens der zweiten Verbindung (15) umfasst und dass die Steuereinheit (6) dazu ausgelegt ist, die Deaktivierung des Desinfektions-Betriebsmodus nach Abschluss der Betätigungsphase des Verschlussorgans (16) im Sinn eines Schließens der zweiten Verbindung (15) automatisch zu befehlen.

6. Rollender Wagen (1) nach einem der Ansprüche 1 bis 5, **dadurch gekennzeichnet, dass** der Wagen (1) ein Geolokalisierungssystem (17), eine Uhr (18) und einen zweiten Speicher (19) zum Speichern von Daten, die von der Uhr (18) und dem Geolokalisierungssystem (17) bereitgestellt werden, umfasst, und dass die Steuereinheit (6) dazu ausgelegt ist, im aktivierten Zustand des Desinfektions-Betriebsmodus das Speichern der von der Uhr (18) und dem Geolokalisierungssystem (17) bereitgestellten Daten in Abhängigkeit von mindestens dem geöffneten/geschlossenen Zustand des Verschlussorgans (16) und/oder vom ein-/ausgeschalteten Zustand der Pumpe (9) zu befehlen.

7. Rollender Wagen (1) nach einem der Ansprüche 1 bis 6, **dadurch gekennzeichnet, dass** der Desinfektionsfluid-Vorratsbehälter (8) mit mindestens einer Heizeinrichtung (20) ausgestattet ist.

8. Rollender Wagen (1) nach einem der Ansprüche 1 bis 7, **dadurch gekennzeichnet, dass** das System (4) zur Bestimmung der Geschwindigkeit des Wagens ein Tachometer ist.

9. Rollender Wagen (1) nach einem der Ansprüche 1 bis 8, **dadurch gekennzeichnet, dass** die erste und zweite Verbindung (14, 15) jeweils mit einem Rückschlagventil (21) ausgestattet sind, das sich zwischen dem Verteilerkreis (10) und der Pumpe (9) für die erste Verbindung (14) und zwischen dem Verteilerkreis (10) und dem Verschlussorgan (16) für die zweite Verbindung (15) erstreckt.

10. Rollender Wagen (1) nach einem der Ansprüche 1 bis 9, **dadurch gekennzeichnet, dass** die erste und zweite Verbindung (14, 15) jeweils mit einem Drucksensor (22) ausgestattet sind, der sich zwischen dem Verteilerkreis (10) und der Pumpe (9) für die erste Verbindung (14) und zwischen dem Verteilerkreis (10) und dem Verschlussorgan (16) für die zweite Verbindung (15) erstreckt und dass die Steuereinheit (6) dazu ausgelegt ist, den Wechsel aus dem aktiven Zustand in den inaktiven Zustand des Desinfektions-Betriebsmodus in Abhängigkeit von den von den Drucksensoren (22) bereitgestellten Daten zu befehlen.

## Claims

1. A wheeled vehicle (1) comprising a chassis (2), wheels (3) for movement on the ground of said chassis (2), a system (4) for determining the speed of the vehicle (1), a memory (5) for storing at least one setpoint speed, a control unit (6) and an on board installation (7) for disinfecting the wheels (3) and the bottom of the chassis (2) of the vehicle (1), said installation (7) comprising a reservoir (8) of disinfecting fluid, a pump (9) for dispensing disinfecting fluid, and a circuit (10) for dispensing fluid equipped at its outlet with nozzles (11), at least some of which are oriented toward the wheels (3) of the vehicle (1), the vehicle (1) comprising a pressurized fluid source (12), a so-called disinfecting operating mode that can be activated/deactivated and a manually actuated member (13) for activating said operating mode, the fluid dispensing circuit (10) being connected, by a first link (14) on which said pump (9) of the installation (7) is arranged, to the disinfecting fluid reservoir (8) and, by a second link (15) equipped with a shutter member (16) mounted so as to move between an open position and a closed position, to the pressurized fluid source (12), **characterized in that** the disinfecting operating mode is an operating mode that can be activated/deactivated based at least on data supplied by the system (4) for determining the speed, and **in that** the control unit (6) is configured so as, in the manually actuated state of the activating member (13), to control the activation of the disinfecting operating mode, when the speed of the vehicle (1) is within a predefined range of setpoint speeds and **in that** the control member (6) is configured so as, in the activated state of the disinfecting operating mode, to command the pump (9) and the shutter member (16) according to a disinfecting cycle comprising at least a first phase of actuating the pump (9) to supply the fluid dispensing circuit (10) with disinfecting fluid and a second phase of actuating the shutter member (16) in the direction of opening of the second link (15) to supply the fluid dispensing circuit (10), which is prefilled with disinfecting fluid, with pressurized fluid, this pressurized fluid serving as fluid for propelling the disinfecting fluid through the nozzles (11).

2. The wheeled vehicle (1) according to claim 1, **characterized in that** the vehicle (1) comprises a second operating mode called unclogging mode of the nozzles (11) that can be activated/deactivated, **in that** the control unit (6) is configured to automatically control the activation or the deactivation of the second operating mode based at least on the data supplied by the system (4) for determining the speed, and **in that** the control unit (6) is configured so as, in the activated state of the second operating mode, to command the opening and the closing of the shutter member (16) according to a predefined sequence.

3. The wheeled vehicle (1) according to claim 2, **characterized in that** the control unit (6) is configured to automatically command the activation of the second operating mode when the speed of the vehicle (1) is greater than a threshold setpoint speed, said threshold setpoint speed being greater than the predefined range of setpoint speeds.

4. The wheeled vehicle (1) according to one of claims 1 to 3, **characterized in that** the control unit (6) is configured so as, in the activated state of the disinfecting operating mode, to automatically command the deactivation of the disinfecting operating mode, when the speed of the vehicle (1) is outside the predefined range of setpoint speeds.

5. The wheeled vehicle (1) according to one of claims 1 to 4, **characterized in that** the disinfecting cycle comprises, after the second phase of actuating the shutter member (16) in the direction of opening of the second link (15), a third phase of actuating the shutter member (16) in the direction of closing of the second link (15), and **in that** the control unit (6) is configured to automatically command the deactivation of the disinfecting operating mode at the end of the phase of actuating the shutter member (16) in the direction of closing of the second link (15).

6. The wheeled vehicle (1) according to one of claims 1 to 5, **characterized in that** the vehicle (1) comprises a geolocation system (17), a clock (18) and a second memory (19) for storing data supplied by the clock (18) and the geolocation system (17) and **in that** the control unit (6) is configured so as, in the activated state of the disinfecting operating mode, to command storing of the data supplied by the clock (18) and the geolocation system (17) based at least on the open/closed state of the shutter member (16) and/or the on/off state of the pump (9).

7. The wheeled vehicle (1) according to one of claims 1 to 6, **characterized in that** the disinfecting fluid reservoir (8) is equipped with at least one heating member (20).

8. The wheeled vehicle (1) according to one of claims 1 to 7, **characterized in that** the system (4) for determining the speed of the vehicle is a tachymeter.

9. The wheeled vehicle (1) according to one of claims 1 to 8, **characterized in that** said first and second links (14, 15) are each equipped with a non-return valve (21) extending between the dispensing circuit (10) and the pump (9) for the first link (14) and between the dispensing circuit (10) and the shutter member (16) for the second link (15).

10. The wheeled vehicle (1) according to one of claims 1 to 9, **characterized in that** said first and second links (14, 15) are each equipped with a pressure sensor (22) extending between the dispensing circuit (10) and the pump (9) for the first link (14) and between the dispensing circuit (10) and the shutter member (16) for the second link (15) and **in that** the control unit (6) is configured to command the passage from the active state to the inactive state of the disinfecting operating mode based on data supplied by said pressure sensors (22).
